# EUROPÄISCHE PATENTANMELDUNG

(11) **EP 1 298 434 A1**
(43) Veröffentlichungstag der Anmeldung: **02.04.2003**
(21) Anmeldenummer: 01402527.4
(22) Anmeldetag: 01.10.2001
(51) Int. Cl.: G01N 33/50, G01N 33/74

(54) **Verfahren zum Auffinden neuer Wirkstoffe mit androgen-ähnlichen Eigenschaften**

(71) Anmelder: Cognis France S.A., 31360 Saint-Martory (FR)
(72) Erfinder: Pauly, Gilles, 54000 Nancy (FR); Gillon, Véronique, 54270 Essey-Les-Nancy (FR); Contet-Audonneau, Jean-Luc, 54130 Saint-Max (FR); Danoux, Louis, 54420 Saulxures-Les-Nancy (FR)
(74) Vertreter: Cabinet HERRBURGER

(57) **Zusammenfassung**

Vorgeschlagen wird ein Verfahren zum Auffinden neuer Wirkstoffe mit androgen-artigen Eigenschaften für die Herstellung von kosmetischen Zubereitungen für Männer, bei man die potentiellen Wirkstoffe mit Kulturen männlicher Fibroblasten in Kontakt bringt und im Verlauf der Wechselwirkung die Menge der gebildeten dermalen Makromolekülen gegenüber einem Standard bestimmt.

## Beschreibung

### Gebiet der Erfindung

Die Erfindung befindet sich auf dem Gebiet der Herrenkosmetik und betrifft ein Verfahren, mit dessen Hilfe sich die Wirksamkeit potentieller Wirkstoffe beurteilen lässt sowie die Verwendung von männlichen Fibroblasten innerhalb dieses Verfahrens.

### Stand der Technik

Die menschliche Haut ist Schauplatz einer Vielzahl von Stoffwechselreaktionen, an denen Androgene beteiligt sind. Typische Beispiele hierfür sind die 5-α-Reduktion von Testosteron (T) zum aktiven Metaboliten Dihydrotestosteron (DHT), die 3-α-Reduktion von DHT, die Umwandlung von schwachen Androgenen wie z.B. Dehydroepiandosteron und Δ4-Andosterondion zu biologisch aktiveren Steroiden, oder die interzellulare Bindung von Androgenen an spezifische Rezeptorproteine. In Haarfollikel und Drüsen finden sich besonders hohe Androgenkonzentrationen. Diese können den Haarwuchs an bestimmten Stellen der Haut stimulieren, beispielsweise in den Achselhöhlen, sie können jedoch bei genetisch vordisponierten Männern auch die Glatzenbildung fördern. Androgene können auch die Größe und die Sekretion von Drüsen beeinflussen und auf diese Weise Störungen im Hautbild, wie beispielsweise Akne oder Seborrhea hervorrufen. Die Lokalisierung von Androgenrezeptoren in den Fibroblasten der menschlichen Haut wird beispielsweise von Liang et al. in **J.Invest.Dermatol. 100, 663-666 (1993)** beschrieben. Die Wirkung von Androgenen auf das Wachstum der Fibroblasten wird üblicherweise durch Flusscytometrie bestimmt. Aus Untersuchungen von Loire et al. **[Biol. of the cell 56, 8989-1992 (1986)]** und Sultan et al. **[Brit.J.Dermatol. 107, 40-46 (1982)]** ist bekannt, dass Dihydrotestosteron die DNA und Proteinverteilung nicht verändert. Daraus kann man ableiten, dass Androgene weder die Proteinsynthese noch die DNA-Produktion induzieren. Die Untersuchung des Einflusses von Testosteron auf die Differenzierung von menschlichen Hautzellen hat gezeigt, dass Testosteron weder das Volumen des Epidermisgewebes, noch die Zellgrösse oder die Zahl der Keratinfilamente beeinflusst, sondern der Abnahme granularer Zellen, Keratohyalingranulen und Keratinosomen entgegenwirkt. Kligman et al. berichten, dass die topische Anwendung von Testosteronpropionat im Achselbereich älterer Männer die Stärke der Epidermis vergrößert **[Adv.Biol.Skin, 6, 177-198 (1965)]**.

Grundsätzlich gilt, dass die Androgenproduktion des Mannes im Alter nachlässt. Ebenfalls ist bekannt, dass die Zellantwort auf Hormone sich im Laufe der Zeit verändert. So konnte gezeigt werden, dass mit dem Alter die Zahl der Bindungsstellen für Androgenzeptoren in den Fibroblasten abnimmt. Detailliertere Studien gehen davon aus, dass diese Änderungen nicht in direktem Zusammenhang mit dem Testosteronlevel stehen, sondern es sich um einen genetisch bedingten Prozess handelt. Damit stellt sich die Frage, ob sich das aus dem Stand der Technik bekannte Wissen über männliche Hormone nicht einsetzen lässt, um ein Modell zu entwickeln, mit dessen Hilfe man neue potentielle Wirkstoffe gerade für die Entwicklung von Herrenkosmetik auf ihre Wirksamkeit testen kann.

Die Aufgabe der Erfindung hat folglich darin bestanden, ein Verfahren zum Auffinden von neuen Wirkstoffen mit androgen-artiger Wirkung für die Herstellung kosmetischen Zubereitungen speziell für die Anwendung im Bereich der Herrenkosmetik zu entwickeln.

### Beschreibung der Erfindung

Gegenstand der Erfindung ist ein Verfahren zum Auffinden neuer Wirkstoffe mit androgen-artigen Eigenschaften für die Herstellung von kosmetischen Zubereitungen für Männer, bei man die potentiellen Wirkstoffe mit Kulturen männlicher Fibroblasten in Kontakt bringt und im Verlauf der Wechselwirkung die Menge der gebildeten dermalen Makromolekülen gegenüber einem Standard bestimmt.

Überraschenderweise wurde gefunden, dass Kulturen männlicher Fibroblasten ideale Systeme darstellen, um die Wirksamkeit von potentiellen neuen Wirkstoffen mit androgener Aktivität für die Herstellung von Herrenkosmetik zu untersuchen. Das Verfahren macht sich dabei die Erkenntnis zu Nutzen, dass aktive Wirkstoffe in Interaktion mit den Fibroblasten die Synthese von dermalen Makromolekülen, wie beispielsweise Hyaluronsäure, Chondroitinsulfat, Dermatansulfat, Keratansulfat, Kollagen oder Elastin stimulieren, während nicht aktive Verbindungen gegenüber dem Blindwert keine oder keine nennenswerte Veränderung der Syntheserate bewirken. Als Standards haben sich dabei männliche Geschlechtshormone bewährt. Durch Messung und Verfolgung des Entstehens der dermalen Makromoleküle und Vergleich gegen den Standard kann also auf die Wirksamkeit der untersuchten Testprodukte zurückgeschlossen werden.

### Durchführung des Verfahrens

Im Sinne des Verfahrens können sowohl embryogene als auch männliche adulte Fibroblasten eingesetzt und kultiviert werden. Unter dem Begriff Fibroblasten sollen auch alle Zellen und Zelltypen verstanden werden, die fibroblastenähnliche Eigenschaften aufweisen, speziell Rezeptoren an ihrer Oberfläche aufweisen, die mit denen der Fibroblasten vergleichbar oder identisch sind. Die Fibroblasten können auch gemeinsam mit anderen Zellen oder Zellinien, speziell aber Hautzellen eingesetzt werden, wie beispielsweise Melanocyten, Langerhans Zellen, Endothelialzellen, Keratinocyten, Neuronen und dergleichen. Als männliche Geschlechtshormone, die als Standards in Frage kommen und gegen die die Aktivität der potentiellen Wirkstoff getestet und beiurteilt werden kann, kommen beispielsweise Testosteron, Dihydrotestosteron, Dehydroepiandrosteron (DHEA) sowie deren Vorstufen und Derivate in Frage. Die Einsatzmenge sowohl der Wirkstoffe als auch der Standards kann jeweils 0,0001 bis 0,001 Gew.-% - bezogen auf die Kulturen - betragen und liegt typisch bei 0,0002 Gew.-%. Die Auswahl der potentiellen Wirkstoffe ist insofern unkritisch, als das System zuverlässig arbeitet, solange diese eine Stimulation der Synthese von dermalen Makromolekülen stimulieren. In der Praxis wird man die Testsubstanzen vorzugsweise aus der Gruppe der Sterole, Peptide, Proteine, Lipide, Zucker und Polysaccharide auswählen, da von diesen die höchste Aktivität in der Stimulation erwartet werden kann. Als Parameter wird die Bildung von dermalen Makromolekülen aus der Gruppe der Glycosaminglycane, Proteoglycane, Glycoproteine und Proteine verfolgt.

Zur konkreten Durchführung des Verfahrens kann beispielsweise wie folgt vorgegangen werden: Zellsuspension menschlicher Fibroblasten (embryonale Zellen oder männlicher adulter Herkunft) werden mit 1 bis 2 mg/ml einer Kollagensuspension vermischt. Anschließend werden die im Kollagengel enthaltenen Fibroblasten zusammen mit einem Nährmedium (2 Gew.-% fötales Kalbsserum, FCS) in Petrischalen (5 ml/Schale) unter Zugabe von 0,0002 Gew.-% des potentiellen Wirkstoffs bzw. von Testosteron bzw. Dihydrotestosteron als Standard kultiviert. Nach einer Inkubationszeit von 7 Tagen bei 27°C und 5 Vol.-% Kohlendioxid in einer mit Wasserdampf gesättigten Atmosphäre werden Biopsien vorgenommen und histologische Schnitte angefertigt. Die Menge an gebildeten Makromolekülen kann nach zwei verschiedenen Verfahren bestimmt werden :
1. Eine allgemeine Bestimmung durch Anfärbung der GAG mit Hilfe von PAS-Alcian Blau (Standardanfärbung) sowie
2. eine spezifische imunohistochemische Charakterisierung einiger Elemente der Matrix durch Behandlung mit Antikörpern, speziell Anti-Chondroitinsulfat, Anti-Keratansulfat, Anti-heparansulfat, Anti-Elastin und Anti-Kollagen (Typ III).

Die Fibroblasten werden bezüglich der Anfärbung mit Hilfe eines Photonenmikroskopes und bezüglich der Histologie mittels eines konfokalen Lasermikroskops untersucht. Zur Bestimmung der Menge an gebildeten dermalen Makromolekülen werden Aufnahmen angefertigt und die Zonen rund um die Fibroblasten ("Perifibroblastische Zonen") untersucht. Die Auszählung erfolgt über durch Bildanalyse, nämlich durch Bestimmung der perifibroblastischen Zonen und des Graulevels. Beide Parameter sind der Synthese an dermalen Makromolekülen direkt proportional. Die Ergebnisse sind in Relation zu den entsprechenden Ergebnissen ohne Zugabe der Wirkstoffe zu bewerten.

### Gewerbliche Anwendbarkeit

Die Synthese der dermalen Makromolekülen stellt ganz allgemein ein empfindliches Messinstrument dafür dar, wie Alter und Umwelteinflüsse die Prozesse in der Haut des Mannes beeinträchtigen. Weitere Gegenstände der Erfindung betreffen daher nicht nur die Verwendung von männlichen Fibroblasten, alleine oder gemeinsam mit anderen Hautzellen, in dem eingangs geschilderten Verfahren zur Auffindung von neuen Wirkstoffen mit androgen-artigen Eigenschaften für die Herstellung von kosmetischen Zubereitungen für Männer, sondern auch ihre Verwendung - wiederum alleine oder zusammen mit anderen Zellen oder Zelllinien, speziell aber Hautzellen -
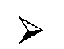 zur Untersuchung von pathologischen Einflüssen auf die Stimulation der Zellsynthese;
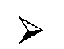 zur Untersuchung von Alterungseinflüssen auf die Stimulation der Zellsynthese;
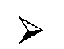 zur Untersuchung von Stresseinflüssen auf die Stimulation der Zellsynthese sowie
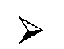 zur Untersuchung des Wirkmechanismus der Stimulation der Synthese von dermalen Makromolekülen durch männliche Geschlechtshormone.

### Beispiele

Wie im Kapitel "Durchführung des Verfahrens" beschrieben, wurden verschiedene Fibroblastenkulturen mit und ohne Zugabe von männlichen Geschlechtshormonen kultiviert und die Synthese der dermalen Makromoleküle verfolgt. Die Ergebnisse sind in Tabelle 1 zusammengefasst. Die Angaben verstehen sich als Zunahme in %-rel bezogen auf den Blindwert, d.h. die Abwesenheit der männlichen Geschlechtshormone. Dargestellt ist der Mittelwert der beiden Messverfahren.

**Tabelle 1**

| Synthese von dermalen Makromolekülen | | | | | | |
|---|---|---|---|---|---|---|
| | 1 | 2 | 3 | 4 | 5 | 6 |
| Hormontyp | T (löslich) | DHT | T | DHT | T | DHT |
| Fibroblasten | E | MA | MA | MA | MA | MA |

| *Zunahme der Synthese an Makromolekülen* | | | | | | |
|---|---|---|---|---|---|---|
| - Chondroitinsulfat | + 23 | | | + 132 | + 537 | + 79 |
| - Keratansulfat | | | | | + 83 | |
| - Heparansulfat | | + 486 | | | | |
| - Kollagen | + 17 | | + 113 | + 1742 | + 654 | + 409 |
| - Elastin | + 17 | | | | | |
| T = Testosteron; DHT = Dihydrotestosteron | | | | | | |
| E = embryonale Fibroblasten; MA = männliche adulte Fibroblasten | | | | | | |

## Patentansprüche

1. Verfahren zum Auffinden neuer Wirkstoffe mit androgen-artigen Eigenschaften für die Herstellung von kosmetischen Zubereitungen für Männer, bei man die potentiellen Wirkstoffe mit Kulturen männlicher Fibroblasten in Kontakt bringt und im Verlauf der Wechselwirkung die Menge der gebildeten dermalen Makromolekülen gegenüber einem Standard bestimmt.

2. Verfahren nach Anspruch 1, **dadurch gekennzeichnet, dass** man menschliche dermale Fibroblasten einsetzt.

3. Verfahren nach den Ansprüchen 1 und/oder 2, **dadurch gekennzeichnet, dass** man die Fibroblasten zusammen mit anderen Zellen oder Zelllinien einsetzt.

4. Verfahren nach mindestens einem der Ansprüche 1 bis 3, **dadurch gekennzeichnet, dass** man die Menge der gebildeten Makromoleküle gegenüber der Aktivität von männlichen Geschlechtshormonen als Standards bestimmt.

5. Verfahren nach Anspruch 4, **dadurch gekennzeichnet, dass** man männliche Geschlechtshormone als Standards einsetzt, die ausgewählt sind aus der Gruppe, die gebildet wird von Testosteron, Dihydrotestosteron, Dehydroepiandrosteron (DHEA) sowie deren Vorstufen und Derivaten.

6. Verfahren nach mindestens einem der Ansprüche 1 bis 5, **dadurch gekennzeichnet, dass** man die Synthese von dermalen Makromolekülen verfolgt, die ausgewählt sind aus der Gruppe, die gebildet wird von Glycosaminglycanen, Proteoglycanen, Glycoproteinen und Proteinen.

7. Verwendung von männlichen Fibroblasten, alleine oder gemeinsam mit anderen Zellen oder Zelllinien, in einem Verfahren nach den Ansprüchen 1 bis 6 zur Auffindung von neuen Wirkstoffen mit androgen-artigen Eigenschaften für die Herstellung von kosmetischen Zubereitungen für Männer.

8. Verwendung von männlichen Fibroblasten, alleine oder gemeinsam mit anderen Zellen oder Zelllinien, in einem Verfahren nach den Ansprüchen 1 bis 6 zur Untersuchung von pathologischen Einflüssen auf die Stimulation der Zellsynthese.

9. Verwendung von männlichen Fibroblasten, alleine oder gemeinsam mit anderen Zellen oder Zelllinien, in einem Verfahren nach den Ansprüchen 1 bis 6 zur Untersuchung von Alterungseinflüssen auf die Stimulation der Zellsynthese.

10. Verwendung von männlichen Fibroblasten, alleine oder gemeinsam mit anderen Zellen oder Zelllinien, in einem Verfahren nach den Ansprüchen 1 bis 6 zur Untersuchung von Stresseinflüssen auf die Stimulation der Zellsynthese.

11. Verwendung von männlichen Fibroblasten, alleine oder gemeinsam mit anderen Zellen oder Zelllinien, in einem Verfahren nach den Ansprüchen 1 bis 6 zur Untersuchung des Wirkmechanismus der Stimulation der Synthese von dermalen Makromolekülen durch männliche Geschlechtshormone.
